(19) Europäisches Patentamt / European Patent Office / Office européen des brevets

(11) **EP 1 872 715 B1**

(12) **EUROPEAN PATENT SPECIFICATION**

(45) Date of publication and mention
of the grant of the patent:
**13.05.2009 Bulletin 2009/20**

(51) Int Cl.:
***A61B 5/00*** (2006.01)

(21) Application number: **06116380.4**

(22) Date of filing: **30.06.2006**

(54) **Method and system for multi-channel biosignal processing**

Verfahren und System für Mehrkanalbiosignal-Verarbeitung

Méthode et système multicanal pour traitement de biosignaux

(84) Designated Contracting States:
**DE NL**

(43) Date of publication of application:
**02.01.2008 Bulletin 2008/01**

(73) Proprietor: **GENERAL ELECTRIC COMPANY**
**Schenectady, NY 12345 (US)**

(72) Inventor: **Uutela, Kimmo**
**00100 Helsinki (FI)**

(74) Representative: **Valkeiskangas, Tapio Lassi**
**Paavali**
**Kolster Oy Ab**
**Iso Roobertinkatu 23**
**P.O. Box 148**
**00121 Helsinki (FI)**

(56) References cited:
**EP-A- 0 470 764       EP-A- 1 424 637**
**WO-A-02/13689**

## Description

BACKGROUND OF THE INVENTION

This invention relates generally to methods for reducing artifacts in an electric signal, and more particularly, to methods and systems for reducing artifacts induced by a Magnetic Resonance Imaging (MRI) system into a biosignal, typically into an electrocardiogram (ECG).

MRI is used to visualize the inside of living beings as well as to detect the amount of bound water in geological structures. It is primarily used for medical imaging. During an MRI scan, a living being is placed in a turbulent electromagnetic environment created by an MRI device. This turbulent electromagnetic environment may be, for example, a rapidly changing magnetic field. Hereinafter, the living being is referred to as a patient. The cardiac condition of a patient placed in the rapidly changing magnetic field needs to be monitored. An ECG signal is a good indicator of the cardiac condition of the patient. Generally, the term 'ECG signal' may refer to a conventional ECG signal or a multi-channel ECG signal. In MRI environment, a single channel of ECG is conventionally monitored. A multichannel ECG may also be used to provide more accurate information of the cardiac condition.

The ECG signal may be monitored for the purpose of both cardiac monitoring and synchronization of the imaging of the patient's cardiac cycle. However, the rapidly changing magnetic field generated by the MRI device induces interference signals into the patient's ECG signal. These interference signals are hereinafter referred to as artifacts. These artifacts may be defined as any unwanted visible effect in the ECG signal created by disturbances in signal processing. For example, disturbances created by the flow of blood or cerebrospinal fluid may be termed as flow artifacts. Further, disturbances created by the interference of the rapidly changing magnetic field generated by the MRI device, with the ECG signal, may be termed as gradient artifacts.

Various methods exist in the art for measuring and extracting the blood flow in a single channel of ECG signal. However, the artifacts induced due to the rapidly changing magnetic field created by the MRI device, are required to be removed from the multiple channels of the ECG signal. Different types of reference data may be used to detect and remove the artifacts. EP-A-1 424 637 discloses a method and apparatus for suppressing interference in an electric signal, such as an ECG signal, in connection with MRI imaging. A separate reference signal is formed, which serves as a reference for identifying artifacts in the ECG signal. The reference signal may be derived from the ECG signal or it may also be an external signal obtained from the MRI device, for example. The reference signal is then used to select artifact-free samples from a high rate sample sequence of the original ECG signal and the selected samples are supplied to a down-sampling process. EP-A-0 470 764 in turn discloses an adaptive filtering system for reducing the electrical noise caused by gradient switching and the like on low powered bio-potential signals. A noise reference signal, related to the electromagnetic fields produced by the MRI system, is processed by a filter having adjustable filter coefficients to produce a filtered noise reference signal. This filtered noise reference signal is subtracted from the bio-potential signal to produce an output signal and the output signal used for adjusting the coefficients of the filter so as to minimize the output signal.

However, the existing methods do not completely remove all the various artifacts. The present invention seeks to alleviate or eliminate this drawback.

BRIEF DESCRIPTION OF THE INVENTION

The objective of the present invention is to bring about a novel mechanism for removing artifacts from a multiple channel biosignal measured from a subject being studied in an MRI device. The present invention further seeks to improve the signal-to-noise ratio of biosignal measurements made in MRI environment. The biosignal is typically a multichannel ECG, although it may also be a multichannel electroencephalogram (EEG), for example.

This objective is achieved with the solution defined in the independent patent claims.

In the method of the invention, reference data indicative of statistical properties of biosignal artifacts is initially collected. Further, multiple channels of a biosignal of a patient, typically an ECG, are measured in a turbulent electromagnetic environment. Several artifacts may be induced into the multiple channels of the biosignal by the turbulent electromagnetic environment. These artifacts are detected using the reference data. Thereafter, parameters for a linear combination of the multiple channels, which is less sensitive to the artifacts than the unprocessed biosignal, are derived and the biosignal is refined using the linear combination defined by the parameters derived. Ideally, the flow and gradient artifacts are removed from the refined biosignal thus obtained.

The system of the invention collects reference data indicative of statistical properties of biosignal artifacts, typically ECG artifacts. The system also measures the multiple channels of the biosignal of a patient in the turbulent electromagnetic environment. Further, the system derives parameters for a linear combination of the multiple channels, which is less prone to the artifacts than the multiple channels, and refines the biosignal by applying the linear combination defined by the parameters to desired signal samples of the multiple channels. As discussed below, the desired signal samples may be subsequent signal samples measured in real time or signal samples measured earlier from the patient. The latter samples may include the samples used to derive the parameters or other samples measured earlier from the patient, i.e. the mechanism of the invention may also be applied off-line.

BRIEF DESCRIPTION OF THE DRAWINGS

FIG. 1 is an exemplary environment where the invention may be practised, in accordance with various embodiment of the invention.

FIG. 2 is a flowchart depicting a method for obtaining a refined ECG signal of a patient located in a turbulent electromagnetic environment, in accordance with various embodiments of the invention.

FIG. 3 is a flowchart depicting a method for obtaining a refined ECG of a patient located in a turbulent electromagnetic environment, in accordance with an embodiment of the invention.

FIG. 4 is an exemplary schematic representation of ECG measurement of a patient in an MRI environment, in accordance with an embodiment of the invention.

FIG. 5 is a flowchart depicting a method for obtaining a refined ECG of a patient located in a turbulent electromagnetic environment, in accordance with an alternative embodiment of the invention.

FIG. 6 is a flowchart depicting a method for obtaining a refined ECG of a patient located in a turbulent electromagnetic environment, in accordance with an alternative embodiment of the invention.

FIG. 7a illustrates two ECG signals of a patient, in accordance with an embodiment of the invention.

FIG. 7b illustrates two refined ECG signals of a patient, in accordance with an embodiment of the invention.

FIG. 8 illustrates a system for obtaining a refined ECG of a patient located in a turbulent electromagnetic environment, in accordance with an embodiment of the invention.

DETAILED DESCRIPTION OF THE INVENTION

Various embodiments of the invention provide a method and a system for reducing artifacts in an electric signal. Specifically, various embodiments of the invention provide methods and systems for reducing artifacts induced by a Magnetic Resonance Imaging (MRI) system into a biosignal, which is typically an electrocardiogram (ECG). In various embodiments of the invention, the artifacts may be MRI gradient artifacts and/or flow artifacts, the flow artifacts being the disturbances created by the flow of the blood or cerebrospinal fluid of a patient being exposed to the magnetic field of the MRI system. Below, the invention is discussed assuming that the biosignal measured from a patient being studied in an MRI device is a multichannel ECG.

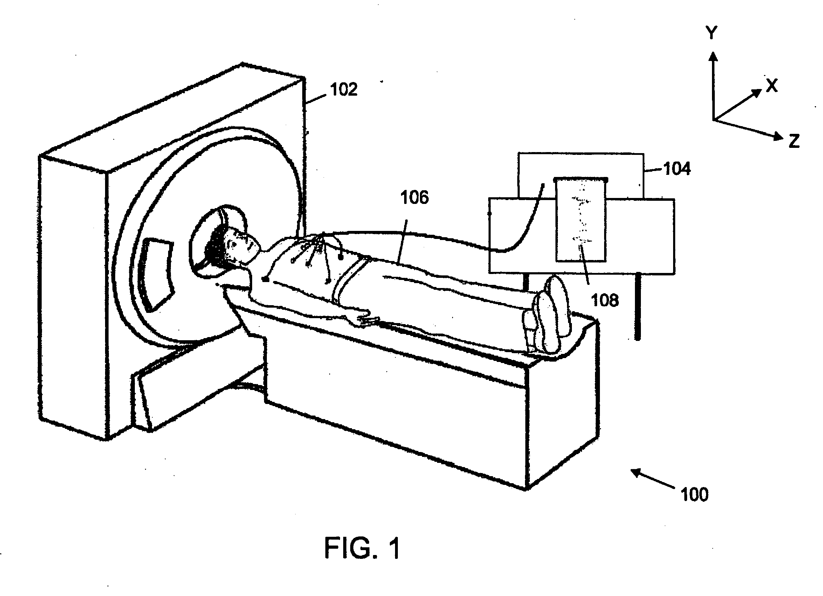

FIG. 1 is an exemplary environment 100, in which various embodiments of the invention may be implemented. Environment 100 includes an MRI device 102 and an ECG-measuring device 104. A patient 106 is placed in a rapidly changing magnetic field produced by MRI device 102.

MRI device 102 includes a main magnet and a plurality of gradient magnets. These are low-strength gradient magnets, as compared to the main magnet. In an embodiment of the invention, the strength of the main magnet is typically between 0.5 tesla and 3 tesla, and the strength of the gradient magnets may be between 18 millitesla and 270 millitesla. In an alternative embodiment of the invention, magnets of different field strengths may be used. The main magnet immerses patient 106 in a stable and intense magnetic field, and the gradient magnets create a turbulent magnetic field.

An ECG signal 108 is measured by ECG-measuring device 104. ECG signal 108 is an indicator of the cardiac condition of patient 106. In an embodiment of the invention, ECG signal 108 is a multi-channel ECG signal.

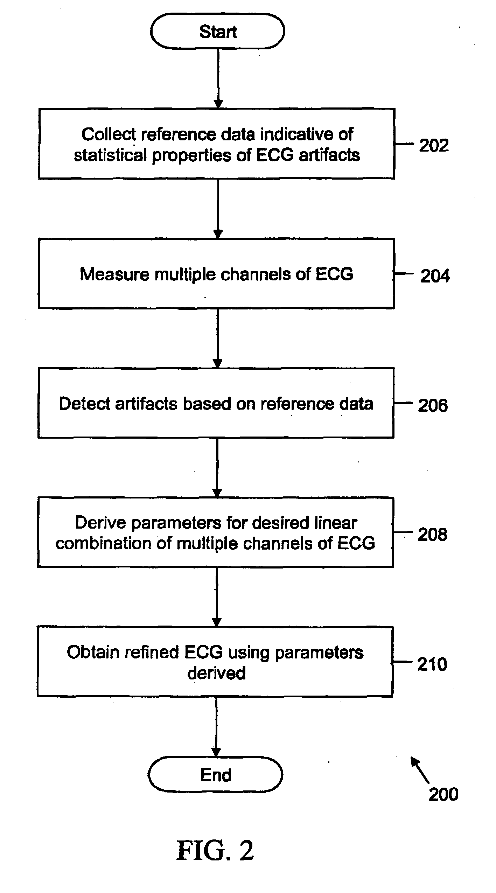

FIG. 2 is a flowchart depicting a method for obtaining a refined ECG of patient 106 located in a turbulent electromagnetic environment, in accordance with various embodiments of the invention. In various embodiments of the invention, the turbulent electromagnetic environment may be a rapidly changing magnetic field. This rapidly changing magnetic field may be created by an MRI device.

At 202, reference data indicative of statistical properties of ECG artifacts is collected. This reference data may be obtained from the ECG signal of patient 106, which is measured in the turbulent electromagnetic environment. The ECG signal of the patient is a multi-channel ECG signal. In an embodiment of the invention, the reference data is collected for each channel of the multi-channel ECG signal over a period of time. The period of time may vary, for example, from a minute to an hour.

At 204, an ECG signal of patient 106 in the turbulent magnetic field is measured in real time. The ECG signal of patient 106 may include several artifacts, including the artifacts induced by the turbulent electromagnetic environment. At 206, the artifacts are detected in each of the multiple channels of the ECG signal measured at 204. In an embodiment of the invention, it is detected that the measured ECG signal contains artifacts. This detection may be performed based on the reference data collected at step 202.

At 208, parameters are derived for a desired linear combination of the multiple channels of the ECG signal, the linear combination having the desired characteristic of being less prone to artifacts than the original ECG. In an embodiment of the invention, the combination may be a linear combination of the multiple channels of the ECG signal that are measured at 204. In another embodiment of the invention, this linear combination may be a weighted linear combination. In an embodiment of the invention, the weight of each channel may depend on several factors, such as the orientation of the electrode configuration with respect to the direction of the flow of blood.

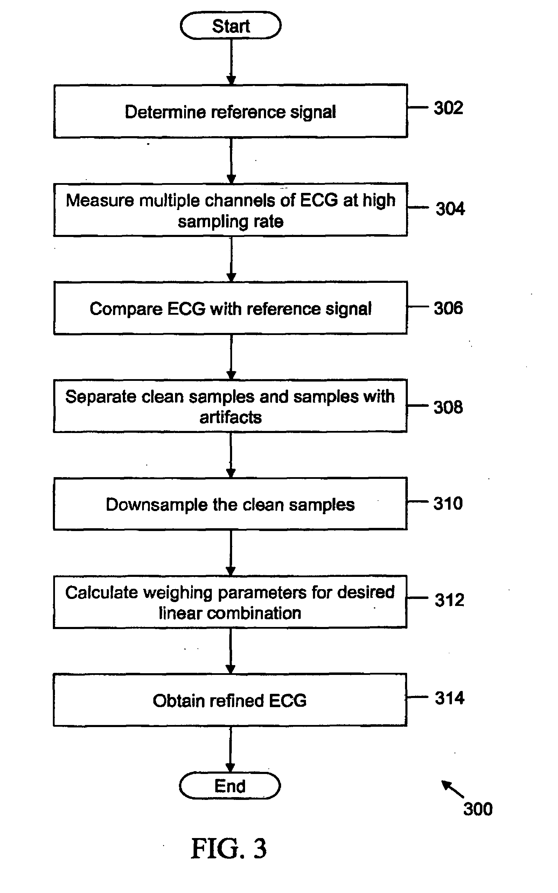

At 210, a refined ECG signal is obtained by applying the linear combination defined by the derived parameters to desired signal values of the channels. Here, the linear combination defined by the parameters may be applied to new and/or old signal samples. When the said linear combination is applied to new signal samples, the said signal samples may be processed similarly as the signal samples based on which the parameters were obtained. The method of obtaining the combination of the multiple channels and the refined ECG signal is explained in conjunction with FIG.3, FIG. 4, FIG.5 and FIG. 6. In various embodiments of the invention, the artifacts induced by the turbulent magnetic field are minimized in the refined ECG signal. The output of step 210 may thus be termed artifact-free ECG.

FIG. 3 is a flowchart depicting a method for obtaining a refined ECG of patient 106 located in a turbulent electromagnetic environment, in accordance with an embodiment of the invention. The refined ECG may be free from MRI gradient artifacts, which may be defined as the interference of the rapidly changing magnetic field produced by the MRI device with the ECG signal of patient 106.

At 302, a reference signal is determined. This is a multi-channel reference signal, where each channel of the reference signal corresponds to one channel of the ECG signal. The reference signal may be determined by using any of the methods already known in the art. For example, in US2004/135571, the reference signal is generated by limiting the slew rate of the original signal. The reference signal serves as a reference for identifying the artifacts in the ECG signal of patient 106. In an embodiment of the invention, the measured ECG signal may serve as the reference signal. In another embodiment of the invention, the reference signal is generated at the same time when the ECG signal is measured.

At 304, the multiple channels of the ECG signal of patient 106 are measured in real time at a high sampling rate. The measured ECG signal may include several artifacts, including the artifacts induced by the turbulent electromagnetic environment. At 306, the ECG signal is compared with the reference signal, to identify the artifacts induced by the turbulent electromagnetic environment. In an embodiment of the invention, a comparison is performed by comparing each channel of the ECG signal with the corresponding channel of the reference signal.

At 308, various clean samples of the ECG signal are separated from the samples of the ECG signal with artifacts, based on the comparison performed at 306. In various embodiments of the invention, clean samples are identified based on the reference signal. Clean samples are samples of the ECG signal that are regarded as being substantially free from artifacts. The process of separating the clean samples from the samples with artifacts is already known in the art. For example, if the difference between the samples and the reference signal is below a predefined limit, then such samples may be marked as clean samples. At 310, the clean samples are downsampled. The process of downsampling may be performed in many ways, which are already known in the art. For example, the downsampling may be carried out by calculating a single average sample from the clean samples. In various embodiments of the invention, the downsampling is performed for most of the channels of the ECG signal.

At 312, weighting parameters for the linear combination are calculated by using the samples of the ECG signal with artifacts. In an embodiment of the invention, a multichannel correlation matrix $\mathbf{A}$ of ECG samples with artifacts is calculated to collect statistical information about the artifacts. The multichannel correlation matrix $\mathbf{A}$ is calculated as

$$\mathbf{A} = \sum_{i=1}^{n} (\mathbf{x}_i - \overline{\mathbf{x}})(\mathbf{x}_i - \overline{\mathbf{x}})^t ,$$

where n is the total number of samples, and $\mathbf{x}_i$ is a column vector with the down-sampled multichannel data of a sample i, and $\overline{\mathbf{x}}$ is the average of all samples, and $()^t$ is the transpose.

The parameters for the linear combination are calculated by taking the biggest eigenvector of matrix A. The eigenvector is then normalized, so that its norm is 1. This normalized eigenvector is represented as '$\mathbf{e}$'. The parameters for the linear combination removing the strongest artifact component are then the projection matrix $\mathbf{P}$ that can be calculated as

$$\mathbf{P} = \mathbf{I} - \mathbf{e}\,\mathbf{e}^t,$$

where I is the identity matrix. Several orthogonal or orthogonalized artifact components can be removed by calculating the projection matrix $\mathbf{P}$ as

$$\mathbf{P} = \mathbf{I} - \mathbf{e}_1\,\mathbf{e}_1^t - \ldots - \mathbf{e}_m\,\mathbf{e}_m^t ,$$

where $e_1 \ldots e_m$ are the artifact components. The projection matrix thus forms the parameters of the linear combination.

In an alternative embodiment of the invention, the statistical information is calculated separately for both samples with artifacts (correlation matrix $\mathbf{A}$) and for clean samples (correlation matrix B). Then, at step 312, the parameters for the linear combination w may be selected so that it minimizes the ratio of the artifact power and clean signal power:

$$\mathbf{w} = \arg\min \frac{\mathbf{w}^t \, \mathbf{A} \, \mathbf{w}}{\mathbf{w}^t \, \mathbf{B} \, \mathbf{w}}$$

At step 314, a refined ECG signal is obtained by applying the linear combination defined by the parameters, either **P** or **w,** to desired signal samples, which may be old or new signal samples. If the said linear combination is applied to new signal samples, steps 302-310 are preferably repeated for the said samples prior to the application of the linear combination, i.e. the new signal samples are preferably processed, prior to the application of the linear combination, similarly as the signal samples based on which the parameters were determined. As also discussed above, the new signal samples are not necessary subsequent signal samples, but the method of the invention may also be applied in off-line state. In an embodiment of the invention, a linear combination that removes the artifact from the ECG signal **x** is calculated as **P x**, or equivalently as **x-e e$^t$ x.** In another embodiment of the invention, one-dimensional linear combination is calculated from the column vector **x** as **w$^t$ x.**

In an alternate embodiment of the invention, the refined ECG is obtained regardless of the exact method of using the reference data.

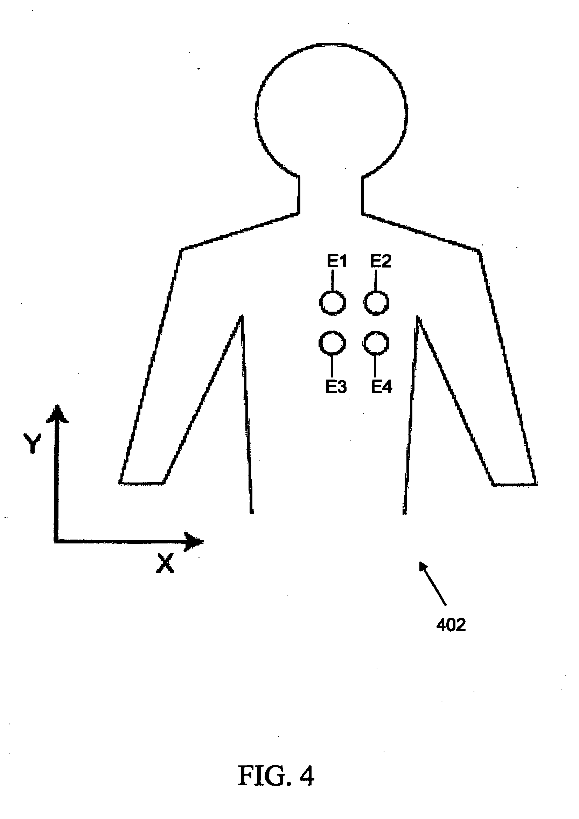

FIG. 4 is an exemplary schematic representation of the ECG measurement of a patient 402 in an MRI environment, in accordance with an embodiment of the invention. The ECG signals are measured across four electrodes, E1, E2, E3 and E4, for patient 402. The four electrodes E1, E2, E3 and E4 are arranged in a rectangular grid pattern at different positions in the body of patient 402. The potential at these locations is measured with reference to one of the electrodes E1, E2, E3 and E4. In an embodiment of the invention, the potential of these locations may be measured against electrode E1. Therefore, the three voltage measurements may be taken as E2 vs E1, E3 vs E1, and E4 vs E1.

In various embodiments of the invention, the voltage measurements for patient 402 are taken in a gradient magnetic field. When the gradient magnetic field produces a potential that increases along the X coordinates, electrodes E2 and E4 are at a higher potential than electrodes E1 and E3. Therefore, the potential pattern in the four electrodes is (0 1 0 1), where 1 represents the similarly changed potential in electrodes E2 and E4. Similarly, when the potential increases along the Y coordinates, electrodes E1 and E2 are at a higher potential than electrodes E3 and E4, and the corresponding potential pattern is (1100).

When the gradient magnetic fields are applied independently along the X and Y coordinates, the strongest subspaces of an artifact correlation matrix A are the voltage patterns measured from the two potential patterns, i.e., (101) for X-oriented artifacts and (0 1 1) for Y-oriented artifacts. When these two subspaces are projected away from the correlation matrix A, the remaining linear combination used to generate the artifact-free subspace is (1/3 1/3 -1/3). This corresponds to a potential pattern (1 -1 -1 1). This implies that, in this case, a combination of the four potentials of the rectangular electrode setup, with weights (1 -1 -1 1), would produce an artifact-free ECG signal.

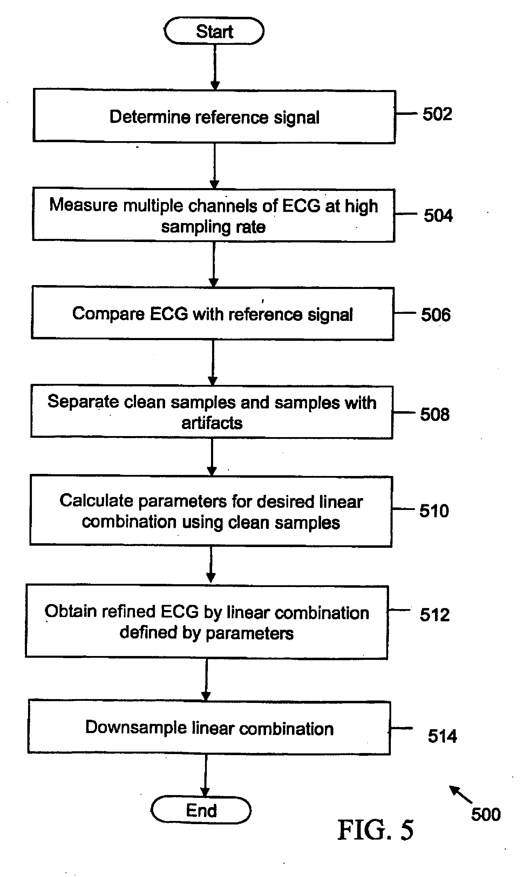

FIG. 5 is a flowchart depicting a method for obtaining a refined ECG of patient 106 located in a turbulent electromagnetic environment, in accordance with an alternative embodiment of the invention. The refined ECG may be free from MRI gradient artifacts.

At 502, a reference signal is determined as explained with reference to FIG. 3. This is a multi-channel reference signal, where each channel of the reference signal corresponds to one channel of the ECG signal. The reference signal serves as a reference for identifying the artifacts in the ECG signal of patient 106.

At 504, the multiple channels of the ECG signal of patient 106 are measured in real time at a high sampling rate. The multiple channels of the ECG signal are measured as explained in conjunction with FIG. 3. At 506, the ECG signal is compared with the reference signal, to identify the artifacts induced by the turbulent electromagnetic environment. In an embodiment of the invention, each channel of the ECG signal is compared with the corresponding channel of the reference signal.

At 508, various clean samples of the ECG signal are separated from the samples of the ECG signal with artifacts, based on the comparison performed at 506. The process of separating the clean samples from those with artifacts is as explained with reference to FIG. 3.

At 510, parameters of the linear combination are calculated by using the clean samples of the ECG signal without downsampling them. The projection vectors are calculated as explained with reference to FIG. 3. Based on the parameters, a linear combination of the ECG channels is obtained such that the linear combination has minimum gradient artifacts. The linear combination of the ECG channels is obtained as explained with reference to FIG. 3.

Further, at 512, a refined ECG is obtained by applying the obtained linear combination of the ECG channels. As discussed above, the linear combination may be applied to old and/or new signal samples.

Finally, the linear combination, in which the signals still have the high sampling rate, is downsampled at 514.

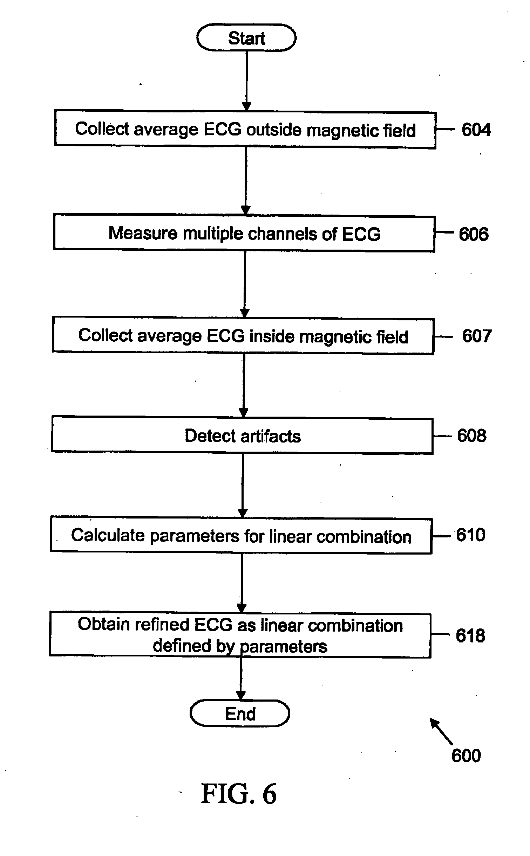

FIG. 6 is a flowchart depicting a method for obtaining a refined ECG of patient 106 located in a turbulent electromagnetic environment, in accordance with an alternative embodiment of the invention. The refined ECG may be free from flow artifacts. Flow artifacts are defined as artifacts induced by the flow of blood or cerebrospinal fluid.

At 604, an average ECG signal outside the turbulent magnetic field is calculated. The average ECG signal is separately calculated for each channel of the multi-channel ECG signal. A plurality of heartbeats are initially detected by using one or more of the multiple channels of the ECG signal. Further, in each of the multiple channels, data portions are selected around each detected heartbeat. An average of the selected data portions of each channel is calculated, to produce the average ECG signal of the specific channel. Alternatively, the average can be calculated in real time by having accumulation buffers. Whenever a heartbeat is detected, selected data from each channel is added to the accumulation buffer of the corresponding channel. The accumulation buffers are then divided by the number of heartbeats to produce the average ECG signal. However, the actual method of calculating the average ECG signal outside the turbulent electromagnetic field is not essential.

At 606, multiple channels of the ECG signal of patient 106 are measured in real time. The multiple channels of the measured ECG signal of patient 106 may include several artifacts, including the artifacts induced by the flow of blood or cerebrospinal fluid. At 607, the average ECG signal inside the turbulent magnetic field is calculated. This process of calculating the average is similar to as explained at 604.

At 608, the gradient artifacts in the multiple channels of the ECG signal are detected and removed. The actual method of detecting the artifacts does not form the core of the invention, and may be performed by any method already known in the art. In an embodiment of the invention, if the gradient artifacts do not pose serious problems for the particular measurement, the ECG signal may be passed unmodified. In an embodiment of the invention, the gradient artifacts may be detected and removed as explained in conjunction with FIG. 3 and FIG. 5.

At 610, parameters for the linear combination are calculated. In an embodiment of the invention, parameters for the linear combination may be spatial projections that reduce the effect of the artifact induced in the ECG signal.

In accordance with various embodiments of the invention, the parameters for the linear combination are obtained by using a matrix formulation. The average ECG signal outside the turbulent magnetic field of one channel (nt time points) is represented as a row vector, and the averages of the different nc channels are stacked to a matrix **'Ao'** with nc rows and nt columns. Similarly, a matrix **'Ai'** is formed by using the average ECG signal inside the turbulent magnetic field. An artifact vector is then calculated by taking the biggest eigenvector of the nc times nc matrix **(Ai-Ao)\*(Ai-Ao)'**. The biggest eigenvector can be an nc-element eigenvector, which is then normalized, so that its norm is 1. This normalized eigenvector is represented as 'e'. The parameters for the linear combination that reduces the artifact can be represented as a projection matrix $P = I - e\ e^t$. Various other methods known in the art may also be used to select the eigenvector **e**.

In another embodiment of the invention, a multichannel correlation matrix A of the artifact may be calculated as

$$A = \sum_{i=1}^{n} (x_i - \bar{x})(x_i - \bar{x})^t$$

Where n is the total number of samples of multichannel ECG signal, $x_i$ are the column vectors containing the differences of the average ECG signals and $\bar{x}$ is the average of all differences. The maximal eigenvector **e** is selected from the correlation matrix **A**. Again, the parameters of the linear combination that reduces the artifact can be represented as a projection matrix $P = I - e\ e^t$.

In an alternative embodiment, statistical information is calculated separately for both the difference of the average ECG (correlation matrix **A**) and for ECG measured outside the turbulent magnetic environment (correlation **B**). Then, the parameters of the linear combination, vector 'w', can be selected so that it minimizes the ratio of the artifact power to clean signal power:

$$w = \arg\min \frac{w^t\ A\ w}{w^t\ B\ w}$$

At step 618, a refined ECG signal is obtained as a linear combination. As in the above embodiments, the linear combination defined by the parameters obtained may be applied to old and/or new signal values and, prior to the application of the said linear combination, the new signal samples may be processed similarly as the signal samples based on which the parameters were obtained. In an embodiment of the invention, the linear combination of data vector **x** reducing the artifacts can be calculated as **P x**, or equivalently as $x - e\ e^t\ x$. In another embodiment of the invention, one-dimensional linear combination is calculated as $w^t\ x$.

In one embodiment of the invention, the effect of the artifact, such as the blood flow, in the ECG signal is evaluated. If **e** is the eigenvector representing the largest eigenvalue of the autocorrelation matrix of the flow artifact, the linear combination representing the strength of the blood flow artifact can be calculated as $e^t\ x$. In another embodiment, vector 'w' can be selected so that it maximizes the ratio of the artifact power and clean signal power, the linear combination representing the strength of the blood flow artifact can be calculated as $w^t\ x$.

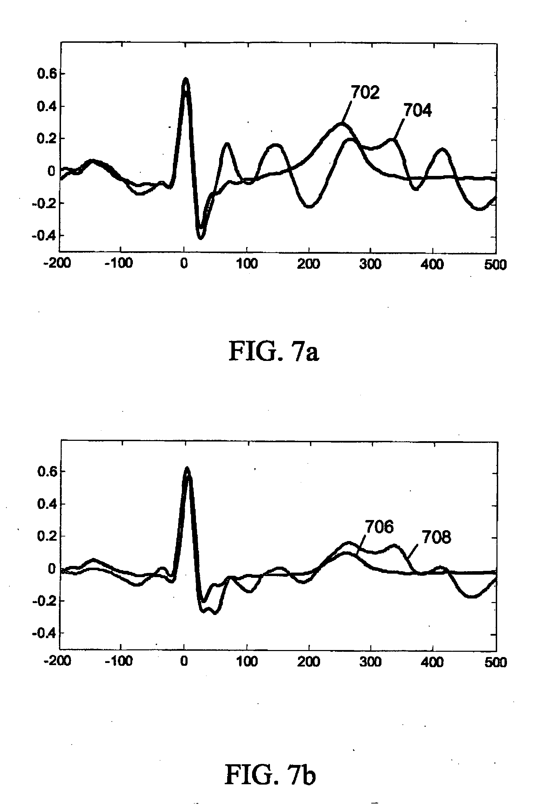

FIG. 7a illustrates two ECG signals of patient 106, in accordance with an embodiment of the invention. A first ECG signal 702 represents a single channel of the multi-channel ECG signal of patient 106 outside the turbulent magnetic field. A second ECG signal 704 represents the channel of the multi-channel ECG signal of patient 106 inside the turbulent magnetic field. The strength of the turbulent magnetic field may vary, for example, from 0T to 5T.

FIG. 7b illustrates two refined ECG signals of patient 106, in accordance with an embodiment of the invention. A first refined ECG signal 706 represents the channel of the multi-channel ECG signal of patient 106 outside the turbulent magnetic field. A second refined ECG signal 708 represents the channel of the multi-channel ECG signal of patient 106 inside the turbulent magnetic field.

The above description assumes that the multichannel biosignal measured from the patient is an ECG. However, the biosignal measured from the patient may also be a multichannel electroencephalogram (EEG). The embodiments of FIGs. 3 and 5 are suitable for various multichannel biosignals, whereas the embodiment of FIG. 6 is primarily for a multi-channel ECG.

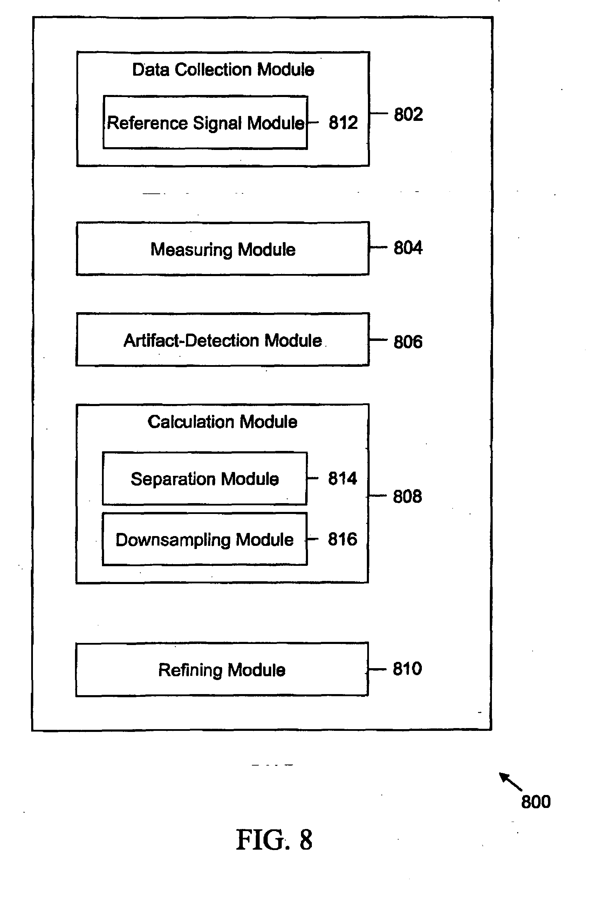

FIG. 8 illustrates a system 800 for obtaining a refined biosignal of patient 106 located in a turbulent electromagnetic environment, in accordance with an embodiment of the invention. System 800 includes a data collection module 802, a measuring module 804, an artifact-detection module 806, a calculation module 808, and a refining module 810.

The data collection module 802 collects reference data indicative of statistical properties of biosignal artifacts, such as ECG artifacts. In various embodiments of the invention, this reference data may include information about the ECG or EEG signal of patient 106 in the turbulent electromagnetic environment. The data collection module 802 includes a reference signal module 812, which determines a reference signal, as explained with reference to FIG. 3. In another embodiment of the invention, the reference data may include the average ECG signal inside the turbulent electromagnetic environment and the average ECG signal outside the turbulent electromagnetic environment.

The measuring module 804 measures the biosignal of patient 106 in real time. The biosignal of patient 106 may include several artifacts, including the artifacts induced by the turbulent electromagnetic environment and the artifacts induced by the flow of blood or cerebrospinal fluid.

The artifact-detection module 806 detects the artifacts induced by the turbulent electromagnetic environment. Calculation module 808 calculates the parameters for the linear combination having the desired characteristics. The calculation module includes a separation module 814 and a downsampling module 816. The separation module 814 separates clean samples from samples with artifacts, based on the comparison performed by the artifact-detection module 806. Clean samples are the samples that are substantially free from artifacts. These clean samples are then forwarded to downsampling module 816, which downsamples the clean samples. The parameters for the linear combination are then calculated as discussed above in connection with FIG. 3.

In another embodiment of the invention, calculation module 808 may calculate the parameters for the linear combination according to one of the embodiments described with reference to FIG. 5 and FIG. 6.

The refining module 810 obtains the refined biosignal by employing the linear combination defined by the parameters obtained from calculation module 808. The refining module 810 may calculate, for example, the linear combination of the ECG signal, which is maximally sensitive to the flow artifact of the ECG signal, and refine the ECG signal using the said linear combination.

Various embodiments of the invention provide a method and a system that reduces artifacts induced by a turbulent electromagnetic environment into an ECG signal. The artifacts induced by the turbulent electromagnetic environment are detected and projected away, to obtain a refined ECG.

A technical effect of the various embodiments of the invention is to reduce the amount of artifacts induced by an MRI system into a biosignal of a patient.

The various embodiments, or the components thereof, may be implemented as a part of a computer system. The computer system may include a computer, an input device, a display unit, and an interface, for example, to access the Internet. It may also include a microprocessor, which may be connected to a communication bus. The computer may include a memory, which may include a Random Access Memory (RAM) and a Read Only Memory (ROM), as well as a storage device, which may be a hard disk drive or a removable storage drive such as a floppy disk drive, an optical disk drive, and so forth. The storage device can also be other similar means of loading computer programs or other instructions into the computer system.

As used herein, the term 'computer' may include any processor-based or microprocessor-based system that includes systems using microcontrollers, reduced instruction set circuits (RISC), application-specific integrated circuits (ASICs), logic circuits, and any other circuit or processor that is capable of executing the functions described herein. The examples given above are exemplary only, and are not intended to limit in any way the definition and/or meaning of the term 'computer'.

The computer system executes a set of instructions that are stored in one or more storage elements, in order to process input data. The storage elements may also hold data or other information, as desired or required, and may be in the form of an information source or a physical memory element in the processing machine.

The set of instructions may include various commands that instruct the processing machine to perform specific operations, such as the processes of the various embodiments of the invention. The set of instructions may be in the form of a software program. The software may be in various forms, such as system software or application software. Further, the software may be in the form of a collection of separate programs, a program module within a larger program, or a portion of a program module. The software may also include modular programming in the form of object-oriented programming. The processing of input data by the processing machine may be in response to in response to user commands, to results of previous processing, or to a request made by another processing machine.

As used herein, the terms 'software' and 'firmware' are interchangeable and include any computer program that is stored in the memory, to be executed by a computer, which includes RAM memory, ROM memory, EPROM memory, EEPROM memory, and non-volatile RAM (NVRAM) memory. The memory types mentioned above are only exemplary and do not limit the types of memory used to store computer programs.

While the invention has been described in terms of various specific embodiments, those skilled in the art will recognize that it can be practised with modification within the scope of the claims.

## Claims

1. A method for obtaining a refined biosignal of a living being (106; 402) located in a turbulent electromagnetic environment (100), the method comprising:

   - collecting (202; 302; 502; 604) reference data indicative of statistical properties of artifacts induced by the turbulent electromagnetic enviromnent (100) into a biosignal of a certain type;
   - measuring (204; 304; 504; 606) multiple channels of a biosignal of the living being (106; 402) in the turbulent electromagnetic environment, wherein the biosignal is of said certain type;
   - detecting (206; 306; 506; 608) artifacts in one or more of the multiple channels using the reference data,

   **characterized by**

   - deriving (208; 308, 310, 312; 508, 510; 610) parameters for a desired linear combination of the multiple channels, wherein the desired linear combination is less prone to the artifacts than the measured multiple channels of the biosignal; and
   - applying (210; 314; 512; 618) a linear combination to desired signal samples of the multiple channels, thereby to obtain the refined biosignal, wherein the linear combination is defined by the parameters derived.

2. The method according to claim 1, wherein the refined biosignal is an electrocardiogram (ECG).

3. The method according to claim 1, wherein the turbulent electromagnetic environment is produced by a Magnetic Resonance Imaging (MRI) device (102).

4. The method according to claim 1, wherein the artifacts are electromagnetic gradient artifacts.

5. The method according to claim 1, wherein the collecting the reference data comprises determining (502) a multi-channel reference signal.

6. The method according to claim 5, wherein the detecting the artifacts comprises comparing (306; 506) the biosignal with the reference signal.

7. The method according to claim 5, wherein the deriving the parameters for the desired linear combination comprises separating (308; 508) clean samples and samples with artifacts from the multiple channels of the biosignal using the reference signal.

8. The method according to claim 7, wherein the measuring comprises sampling (304) the multiple channels at a high sampling rate and the deriving the parameters for the desired linear combination comprises downsampling (310) the clean samples.

9. The method according to claim 7, wherein the deriving the parameters for the desired linear combination comprises using the samples with artifacts to determine the parameters.

10. The method according to claim 1, wherein the artifacts are flow artifacts.

11. The method according to claim 1, wherein the reference data comprises one or more averaged channels of the biosignal measured in the turbulent electromagnetic environment (100), and wherein the biosignal is an ECG.

12. The method according to claim 1, wherein the reference data comprises one or more averaged channels of the biosignal measured (604) outside the turbulent electromagnetic environment (100), and wherein the biosignal is an ECG.

13. The method according to claim 1, wherein the deriving the parameters for the desired linear combination comprises calculating (312) weights of the multiple channels depending on the artifacts.

14. The method according to claim 1, wherein deriving the parameters for the desired linear combination comprises identifying linear projections for differentiating the artifacts.

15. The method according to claim 1, wherein the deriving the parameters comprises identifying a component of the biosignal, the component being maximally sensitive to flow artifact of the biosignal, and wherein the biosignal is an ECG:

**16.** A system (800) for obtaining a refined biosignal of a living being (106; 402) located in a turbulent electromagnetic environment (100), the system comprising:

- a data collection module (802) for collecting reference data indicative of statistical properties of artifacts induced by the turbulent electromagnetic environment (100) into a biosignal of a certain type;
- a measuring module (804) for measuring multiple channels of a biosignal of the living being (106; 402) in the turbulent electromagnetic environment (100), wherein the biosignal is of said certain type;
- an artifact detection module (806) for detecting artifacts in one or more of the multiple channels using the reference data,

**characterised in that** the system further comprises:

- a calculation module (808) for deriving parameters for a desired linear combination of the multiple channels, the linear combination being less prone to artifacts than the measured multiple channels of the biosignal; and
- a refining module (810) for applying a linear combination to desired signal samples of the multiple channels, thereby to obtain the refined biosignal, wherein the linear combination is defined by the parameters derived by the calculation module (808).

**17.** The system according to claim 16, wherein the refined biosignal is an electrocardiogram (ECG).

**18.** The system according to claim 16, wherein the turbulent electromagnetic enviromnent is produced by a Magnetic Resonance Imaging (MRI) device (102).

**19.** The system according to claim 16, wherein the artifacts are electromagnetic gradient artifacts.

**20.** The system according to claim 16, wherein the data collection module (802) comprises a reference signal module (812) for determining a multi-channel reference signal.

**21.** The system according to claim 20, wherein the calculation module (808) comprises a separation module (814) for separating clean samples and samples with artifacts from the multiple channels of the biosignal using the reference signal.

**22.** The system according to claim 21, wherein the calculation module (808) comprises a downsampling module (816) for downsampling the clean samples.

**23.** The system according to claim 16, wherein the artifacts are flow artifacts.

**24.** The system according to claim 16, wherein the reference data comprises one or more averaged channels of the biosignal measured in the turbulent electromagnetic environment (100), and wherein the biosignal is an ECG.

**25.** The system according to claim 16, wherein the reference data comprises one or more averaged channels of the biosignal measured outside the turbulent electromagnetic environment (100), and wherein the biosignal is an ECG.

**Patentansprüche**

**1.** Verfahren zum Erhalten eines verfeinerten Biosignals eines Lebewesens (106; 402), das in einer turbulenten elektromagnetischen Umgebung (100) angeordnet ist, welches Verfahren folgende Schritte umfasst:

- Sammeln (202; 302; 502; 604) von Referenzdaten, die die statistischen Eigenschaften von Artefakten anzeigen, die durch die turbulente elektromagnetische Umgebung (100) in ein Biosignal eines bestimmten Typs induziert wurden;
- Messen (204; 304; 504; 606) mehrerer Kanäle eines Biosignals des Lebewesens (106; 402) in der turbulenten elektromagnetischen Umgebung, wobei das Biosignal dieses bestimmten Typs ist;
- Detektieren (206; 306; 506; 608) von Artefakten in einem oder mehreren der vielen Kanäle unter Verwendung der Referenzdaten, **gekennzeichnet durch**
- Herleiten (208; 308, 310, 312; 508; 510; 610) von Parametern für eine gewünschte lineare Kombination der vielen Kanäle, wobei die gewünschte lineare Kombination weniger anfällig gegenüber den Artefakten ist, als die gemessenen vielen Kanäle des Biosignals; und
- Anwenden (210; 314; 512; 618) einer linearen Kombination auf die gewünschten Signalmuster der vielen Kanäle, wobei ein verfeinertes Biosignal erhalten wird, in dem die lineare Kombination **durch** die erhaltenen Parameter definiert ist.

**2.** Verfahren nach Anspruch 1, indem das verfeinerte Biosignal ein Elektrokardiogramm (EKG) ist.

**3.** Verfahren nach Anspruch 1, in welchem die turbulente elektromagnetische Umgebung erzeugt wird durch eine Kernspinntomographie (MRI) Einrichtung (102).

**4.** Verfahren nach Anspruch 1, in welcher die Artifakte Artefakte eines elektromagnetischen Gradienten sind.

**5.** Verfahren nach Anspruch 1, bei dem das Sammeln der Referenzdaten die Festlegung (502) eines Multikanalreferenzsignals umfasst.

**6.** Verfahren nach Anspruch 5, bei dem das Detektieren der Artefakte das Vergleichen (306; 506) des Biosignals mit dem Referenzsignal umfasst.

**7.** Verfahren nach Anspruch 5, bei welchem das Ableiten der Parameter für die gewünschte lineare Kombination das Separieren (308; 508) von sauberen Mustern und Mustern mit Artefakten aus den vielen Kanälen des Biosignals unter Verwendung des Referenzsignals umfasst.

**8.** Verfahren nach Anspruch 7, bei welchem das Messen das Abtasten (304) der vielen Kanäle mit einer hohen Abtastrate und das Ableiten von Parametern für die gewünschte lineare Kombination das Heruntertakten (310) der sauberen Muster umfasst

**9.** Verfahren nach Anspruch 7, bei welchem das Ableiten der Parameter für die gewünschte lineare Kombination die Verwendung der Muster mit Artefakten zur Bestimmung der Parameter umfasst.

**10.** Verfahren nach Anspruch 1, bei welchem die Artefakte Flussartefakte sind.

**11.** Verfahren nach Anspruch 1, bei welchem die Referenzdaten einen oder mehrere gemittelte Kanäle des Biosignals umfassen, welche in der turbulenten elektromagnetischen Umgebung (100) gemessen wurden, und in welchem das Biosignal ein EKG ist.

**12.** Verfahren nach Anspruch 1, bei welchem die Referenzdaten einen oder mehrere gemittelte Kanäle des Biosignals umfassen, die außerhalb der turbulenten elektromagnetischen Umgebung (100) gemessen (604) wurden, bei welchen das Biosignal ein EKG ist.

**13.** Verfahren nach Anspruch 1, bei welchem das Ableiten der Parameter für die gewünschte lineare Kombination das Errechnen (312) der Gewichte der vielen Kanäle in Abhängigkeit von den Artefakten umfasst.

**14.** Verfahren nach Anspruch 1, bei welchem das Ableiten der Parameter für die gewünschte lineare Kombination die Identifizierung linearer Projektionen für die Unterscheidung der Artefakte umfasst.

**15.** Verfahren nach Anspruch 1, bei welchem das Ableiten der Parameter die Identifizierung einer Komponente des Biosignals umfasst, welche Komponente höchst sensitiv ist für Flussartefakte des Biosignals ist, und bei welchem das Biosignal ein EKG ist.

**16.** System (800) zum Erhalten eines verfeinerten Biosignals eines Lebewesens (106; 402), welches sich in einer turbulenten elektromagnetischen Umgebung (100) befindet, welches System folgende Merkmale umfasst:

- ein Datensammelmodul (802) zum Sammeln von Referenzdaten, die statistische Eigenschaften von Artefakten anzeigen, die durch die turbulente elektromagnetische Umgebung (100) in einem Biosignal eines bestimmten Typs induziert werden;
- ein Messmodul (804) zum Messen vieler Kanäle eines Biosignals des Lebewesens (106; 402) in der turbulenten elektromagnetischen Umgebung (100), bei welchem das Biosignal dieses bestimmten Typs ist;
- ein Artefakterkennungsmodul (806) zum Erkennen von Artefakten in einem oder mehreren der vielen Kanäle unter Verwendung der Referenzdaten, **dadurch gekennzeichnet, dass** das System weiterhin folgende Merkmale umfasst:
- ein Rechenmodul (808) zum Ableiten von Parametern für eine gewünschte lineare Kombination der vielen Kanäle, welche lineare Kombination weniger anfällig für Artefakte ist als die gemessenen vielen Kanäle des Biosignals; und
- ein Verfeinerungsmodul (810) zum Anwenden einer linearen Kombination auf die gewünschten Signalmuster der vielen Kanäle, um dabei das verfeinerte Biosignal zu erhalten, wobei die lineare Kombination definiert ist durch die Parameter, die von dem Rechenmodul (808) erhalten werden.

**17.** System nach Anspruch 16, bei welchem das verfeinerte Biosignal ein Elektrokardiogramm (EKG) ist.

**18.** System nach Anspruch 16, bei welchem die turbulente elektromagnetische Umgebung durch eine Kernspinresonanzeinrichtung (MRI) (102) erzeugt wird.

**19.** System nach Anspruch 16, bei welchem die Artefakte Artefakte eines elektromagnetischen Gradienten sind.

**20.** System nach Anspruch 16, bei welchem das Datensammelmodul (802) ein Referenzsignalmodul (812) umfasst, um ein Multikanalreferenzsignal festzulegen.

**21.** System nach Anspruch 20, bei welchem das Rechenmodul (808) ein Separationsmodul (814) umfasst, um saubere Muster und Muster mit Artefakten von den vielen Kanälen des Biosignals zu separieren unter Verwendung des Referenzsignals.

22. System nach Anspruch 21, bei welchem das Rechenmodul (808) ein Heruntertaktungsmodul (816) zum Heruntertakten der sauberen Muster umfasst.

23. System nach Anspruch 16, bei welchem die Artefakte Flussartefakte sind.

24. System nach Anspruch 16, bei welchem die Referenzdaten einen oder mehrere gemittelte Kanäle des Biosignals umfassen, welches in der turbulenten elektromagnetischen Umgebung (100) gemessen wurde, und bei welchem das Biosignal ein EKG ist.

25. System nach Anspruch 16, bei welchem die Referenzdaten einen oder mehrere gemittelte Kanäle des Biosignals umfassen, welches außerhalb der turbulenten elektromagnetischen Umgebung (100) gemessen wurde, und bei welchem das Biosignal ein EKG ist.

**Revendications**

1. Procédé pour obtenir un biosignal affiné d'un être vivant (106 ; 402) situé dans un environnement électromagnétique turbulent (100), le procédé comprenant les étapes consistant à :

> - collecter (202; 302 ; 502 ; 604) des données de référence reflétant des propriétés statistiques des artefacts induits par l'environnement électromagnétique turbulent (100) dans un biosignal de type défini ;
> - mesurer (204 ; 304 ; 504 ; 606) des canaux multiples d'un biosignal de l'être vivant (106; 402) étant situé dans l'environnement électromagnétique turbulent, le biosignal étant dudit type défini ;
> - détecter (206; 306 ; 506; 608) des artefacts dans un ou plusieurs des canaux multiples en utilisant les données de référence,

> **caractérisé en ce que** le procédé comprend en outre les étapes consistant à :

> - dériver (208 ; 308, 310, 312 ; 508, 510 ; 610) des paramètres afin de réaliser une combinaison linéaire désirée des canaux multiples, la combinaison linéaire désirée étant moins sensible aux artefacts que les canaux multiples mesurés du biosignal ; et
> - appliquer (210 ; 314 ; 512 ; 618) une combinaison linéaire à des échantillons de signal désirés des canaux multiples, pour obtenir de ce fait le biosignal affiné, la combinaison linéaire étant définie par les paramètres dérivés.

2. Procédé selon la revendication 1, dans lequel le biosignal affiné est un électrocardiogramme (ECG).

3. Procédé selon la revendication 1, dans lequel l'environnement électromagnétique turbulent est produit par un dispositif (102) d'imagerie par résonnance magnétique (MRI).

4. Procédé selon la revendication 1, dans lequel les artefacts sont des artefacts électromagnétiques de gradient.

5. Procédé selon la revendication 1, dans lequel l'étape consistant à collecter des données de référence comprend une étape consistant à déterminer (502) un signal de référence multivoie.

6. Procédé selon la revendication 5, dans lequel l'étape consistant à détecter des artefacts comprend une étape consistant à comparer (306 ; 506) le biosignal avec le signal de référence.

7. Procédé selon la revendication 5, dans lequel l'étape consistant à dériver les paramètres pour obtenir la combinaison linéaire désirée comprend une étape consistant à séparer (308 ; 508) les échantillons sains et les échantillons avec des artefacts à partir des canaux multiples du biosignal en utilisant le signal de référence.

8. Procédé selon la revendication 7, dans lequel l'étape consistant à mesurer comprend une étape consistant à échantillonner (304) les canaux multiples à un taux d'échantillonnage élevé et l'étape consistant à dériver les paramètres pour obtenir la combinaison linéaire désirée comprend une étape consistant à sous-échantillonner (310) les échantillons sains.

9. Procédé selon la revendication 7, dans lequel l'étape consistant à dériver les paramètres pour obtenir la combinaison linéaire désirée comprend l'étape consistant à utiliser les échantillons avec des artefacts pour déterminer les paramètres.

10. Procédé selon la revendication 1, dans lequel les artefacts sont des artefacts de flux.

11. Procédé selon la revendication 1, dans lequel les données de référence comprennent un ou plusieurs canaux ramenés à une moyenne du biosignal mesuré dans l'environnement électromagnétique turbulent (100), et dans lequel le biosignal est un ECG.

12. Procédé selon la revendication I, dans lequel les données de référence comprennent un ou plusieurs canaux ramenés à une moyenne du biosignal mesuré (604) à l'extérieur de l'environnement électromagnétique turbulent (100), et dans lequel le biosignal est un ECG.

13. Procédé selon la revendication 1, dans lequel l'étape consistant à dériver des paramètres pour obtenir la combinaison linéaire désirée comprend une étape consistant à calculer (312) les pondérations des canaux multiples selon les artefacts.

14. Procédé selon la revendication 1, dans lequel l'étape consistant à dériver les paramètres pour obtenir la combinaison linéaire désirée comprend une étape consistant à identifier les projections linéaires pour différencier les artefacts.

15. Procédé selon la revendication 1, dans lequel l'étape consistant à dériver les paramètres comprend une étape consistant à identifier une composante du biosignal, la composante étant sensible au maximum à l'artefact de flux d'écoulement du biosignal, et dans lequel le biosignal est un ECG.

16. Système (800) pour obtenir un biosignal affiné d'un être vivant (106 ; 402) étant situé dans un environnement électromagnétique turbulent (100), le système comprenant :

   - un module de collecte de données (802) pour collecter des données de référence reflétant des propriétés statistiques des artefacts induits par l'environnement électromagnétique turbulent (100) dans un biosignal de type défini ;
   - un module de mesure (804) pour mesurer des canaux multiples d'un biosignal de l'être vivant étant (106 ; 402) dans l'environnement électromagnétique turbulent (100), le biosignal étant dudit type défini ;
   - un module de détection d'artefacts (806) pour détecter des artefacts dans un ou plusieurs des canaux multiples en utilisant les données de référence,

   **caractérisé en ce que** le système comprend en outre :

   - un module de calcul (808) pour dériver des paramètres afin de réaliser une combinaison linéaire désirée des canaux multiples, la combinaison linéaire désirée étant moins sensible aux artefacts que les canaux multiples mesurés du biosignal ; et
   - un module d'affinage (810) pour appliquer une combinaison linéaire à des échantillons de signal désirés des canaux multiples, pour obtenir de ce fait le biosignal affiné, la combinaison linéaire étant définie par les paramètres dérivés par le module de calcul (808).

17. Système selon la revendication 16, dans lequel le biosignal affiné est un électrocardiogramme (ECG).

18. Système selon la revendication 16, dans lequel l'environnement électromagnétique turbulent est produit par un dispositif (102) d'imagerie par résonance magnétique (MRI).

19. Système selon la revendication 16, dans lequel les artefacts sont des artefacts électromagnétiques de gradient.

20. Système selon la revendication 16, dans lequel le module de collecte de données (802) comprend un module de signal de référence (812) pour déterminer un signal de référence multivoie.

21. Système selon la revendication 20, dans lequel le module de calcul (808) comprend un module de séparation (814) pour séparer les échantillons sains et les échantillons avec des artefacts à partir des canaux multiples du biosignal en utilisant le signal de référence.

22. Système selon la revendication 21, dans lequel le module de calcul (808) comprend un module de sous-échantillonnage (816) pour sous-échantillonner les échantillons sains.

23. Système selon la revendication 16, dans lequel les artefacts sont des artefacts de flux.

24. Système selon la revendication 16, dans lequel les données de référence comprennent un ou plusieurs canaux ramenés à une moyenne du biosignal mesuré dans l'environnement électromagnétique turbulent (100), et dans lequel le biosignal est un ECG.

25. Système selon la revendication 16, dans lequel les données de référence comprennent un ou plusieurs canaux ramenés à une moyenne du biosignal mesuré à l'extérieur de l'environnement électromagnétique turbulent (100), et dans lequel le biosignal est un ECG.

FIG. 1

```
                    ┌─────────────┐
                    │    Start    │
                    └─────────────┘
                           │
                           ▼
        ┌──────────────────────────────────────┐
        │  Collect reference data indicative of  │──── 202
        │  statistical properties of ECG artifacts│
        └──────────────────────────────────────┘
                           │
                           ▼
        ┌──────────────────────────────────────┐
        │    Measure multiple channels of ECG    │──── 204
        └──────────────────────────────────────┘
                           │
                           ▼
        ┌──────────────────────────────────────┐
        │    Detect artifacts based on reference data  │──── 206
        └──────────────────────────────────────┘
                           │
                           ▼
        ┌──────────────────────────────────────┐
        │    Derive parameters for desired linear  │──── 208
        │   combination of multiple channels of ECG │
        └──────────────────────────────────────┘
                           │
                           ▼
        ┌──────────────────────────────────────┐
        │    Obtain refined ECG using parameters │──── 210
        │              derived                   │
        └──────────────────────────────────────┘
                           │
                           ▼
                    ┌─────────────┐
                    │     End     │
                    └─────────────┘                ↖ 200
```

## FIG. 2

```
                        ┌──────────┐
                        │  Start   │
                        └────┬─────┘
                             │
                             ▼
       ┌────────────────────────────────────────┐
       │        Determine reference signal       │──── 302
       └────────────────────┬───────────────────┘
                             │
                             ▼
       ┌────────────────────────────────────────┐
       │  Measure multiple channels of ECG at high │──── 304
       │              sampling rate               │
       └────────────────────┬───────────────────┘
                             │
                             ▼
       ┌────────────────────────────────────────┐
       │      Compare ECG with reference signal   │──── 306
       └────────────────────┬───────────────────┘
                             │
                             ▼
       ┌────────────────────────────────────────┐
       │   Separate clean samples and samples with │──── 308
       │                 artifacts                │
       └────────────────────┬───────────────────┘
                             │
                             ▼
       ┌────────────────────────────────────────┐
       │        Downsample the clean samples      │──── 310
       └────────────────────┬───────────────────┘
                             │
                             ▼
       ┌────────────────────────────────────────┐
       │  Calculate weighing parameters for desired │──── 312
       │             linear combination          │
       └────────────────────┬───────────────────┘
                             │
                             ▼
       ┌────────────────────────────────────────┐
       │             Obtain refined ECG           │──── 314
       └────────────────────┬───────────────────┘
                             │
                             ▼
                        ┌──────────┐
                        │   End    │
                        └──────────┘
                                              ↖ 300
```

## FIG. 3

FIG. 4

**FIG. 5**

Flowchart 500:

- Start
- Determine reference signal — 502
- Measure multiple channels of ECG at high sampling rate — 504
- Compare ECG with reference signal — 506
- Separate clean samples and samples with artifacts — 508
- Calculate parameters for desired linear combination using clean samples — 510
- Obtain refined ECG by linear combination defined by parameters — 512
- Downsample linear combination — 514
- End

FIG. 6

FIG. 7a

FIG. 7b

## Data Collection Module

Reference Signal Module — 812 — 802

Measuring Module — 804

Artifact-Detection Module — 806

## Calculation Module

Separation Module — 814

— 808

Downsampling Module — 816

Refining Module — 810

800

# FIG. 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1424637 A
- EP 0470764 A
- US 20040135571 A